# EUROPEAN PATENT SPECIFICATION

(11) **EP 1 486 209 B1**
(45) Date of publication and mention of the grant of the patent: **18.06.2008**
(21) Application number: 04020016.4
(22) Date of filing: 20.07.1999
(51) Int. Cl.: A61K 31/57, A61P 1/00, A61K 9/02, A61K 9/32

(54) **Pharmaceutical formulations for the treatment of inflammatory bowel disease**
Pharmazeutische Zusammensetzung enthaltend Beclomethason-Dipropional zur Behandlung entzündlicher Darmerkrankungen
Formulations Pharmaceutiques servant à traiter la maladie intestinale inflammatoire

(30) Priority: 28.07.1998 IT MI981741
(43) Date of publication of application: 15.12.2004
(62) Divisional of application: 99940025.2
(73) Proprietor: CHIESI FARMACEUTICI S.p.A., I-43100 Parma (IT)
(72) Inventor: Chiesi, Paolo, 43100 Parma (IT); Musa, Rossella, 43100 Parma (IT); Bernini, Eva, 43100 Parma (IT); Castiglione, Gian Nicola, 43100 Parma (IT)
(74) Representative: Banfi, Paolo

(56) References cited:
- US-A- 4 350 690
- CAMPIERI ET AL: ALIMENT.PHARMACOL.THER,, vol. 12, no. 4, April 1998 (1998-04), - 1998 pages 361-366, XP002299658

## Description

The invention relates to pharmaceutical compositions for rectal administration to be used for the treatment of Inflammatory Bowel Disease (IBD).

The invention more particularly relates to the use of a topically active corticosteroid, i.e. beclomethasone dipropionate, in the following also referred to as BDP, for the preparation of pharmaceutical compositions for the treatment of Inflammatory Bowel Diseases such as Ulcerative Colitis (UC) and Crohn's Disease (CD).

IBD is an inflammatory disorder of unknown cause which is characterized by a chronic relapsing - remitting course, in which relapses and remissions alternate. IBD's have increased in incidence, in particular in the Northern areas of Western Countries.

UC is characterized by an inflammatory process confined to the most superficial layer of the intestinal wall (submucosa is saved) primarily involving the colonic mucosa. One of the most frequent localizations is the rectal ampulla from which the inflammatory process can extend to the colon, but not beyond the ileo-cecal valve.

CD is yet another type of inflammatory disease of unknown etiology which mainly affects the distal ileum, but which may occur in any part of the bowel. Contrary to what is observed in UC, the inflammatory process is of "segmental" type (affected portions alternate to health areas) and it affects all intestinal layers.

The medical treatment of said diseases is based on the use of immunosuppressive and anti-inflammatory drugs.

The most effective treatment relies on the use by oral or parenteral route of corticosteroids such as prednisolone, as they possess both immunosuppressive and antiinflammatory properties. However, their use is limited due to the possible systemic side effects (such as oedema, hypertension) and inhibitory effects on adrenocorticotropic hormone (ACTH) with consequent reduction of cortisol production by the adrenal glands. In order to avoid such consequences, the advised treatment provides for an initial high doses for a few weeks, followed by the gradual reduction to maintenance dosage, which should however still be the minimal one able of controlling the clinical symptoms.

Corticosteroids with high topical activity but low systemic bioavailability have been therefore developed; said drugs maintain an effectiveness comparable to that of the systemic ones, even though they show a reduced absorption and therefore a marked decrease in the above mentioned adverse effects.

By virtue of their selective action on the affected intestinal mucosa and the high degree of first-pass metabolism in the liver, with a resulting reduction of the systemic absorption, these novel topical corticosteroids have high therapeutical effectiveness with poor incidence of both systemic and endocrine side effects.

The effectiveness of topical corticosteroids, administered in the form of enemas, in distal UC has been widely demonstrated and more recently such treatment has been also proved to be useful in patients affected by CD.

Beclomethasone dipropionate is a potent, well tolerated topical corticosteroid which has successfully been used in the treatment of distal UC. Although being safe and effective, it has only been used in the form of galenic or extemporarily compounded preparations, as no ready-to-use formulations of suitable pharmaceutical characteristics have been commercially made available so far.

The present invention relates to ready-to-use pharmaceutical formulations for the administration of beclomethasone dipropionate (BDP) at the intestinal site, for use in the treatment of IBD.

Accordingly, the invention provides a physically and chemically stable enema formulation, having a small volume of administration (60 ml) and comprising 3 mg of BDP as active ingredient. Said composition is arranged so that the BDP micronized particles remain dispersed in an aqueous medium containing antimicrobials such as benzyl alcohol, methyl and propyl *p*-hydroxybenzoates, buffering salts, agents for adjusting salinity, such as sodium chloride, surfactants and/or wetting agents, such as cetostearyl alcohol, polysorbate 20 and sorbitan monolaurate and characterized by the use of xanthan gum as suspending and thickening agent.

The choice of the excipients proved to be particularly critical in order to reach the following objectives:
a) homogeneity of the active ingredient, at the low concentration of the formulation (0.005 w/v);
b) optimised fluidity and physical stability of the suspension;
c) absence of surface interactions such as to cause crystalline growth;
d) long-term chemical stability of the active ingredient;
e) local tolerability (pH near neutrality and isotonicity);

In the treatment of Ulcerative Colitis, for an effective therapeutical action to be exerted, the active ingredient administered rectally should uniformly spread up to the splenic flexure and further up.

Most of the BDP containing enema formulations reported until now are extemporary compounded preparations. Otherwise they envisage the use of lower doses and/or larger administration volumes.
- Levine et al (Gastroenterology 1985, 88, 1473), in a clinical study lasting 30 months, disclosed the use of formulations containing 0.5 - 4.0 mg in a volume varied from 50 to 400 ml to be administered daily or b.i.d.
- Mulder et al (Eur. J. Gastroenterol & Hepatol 8, 549-553, 1996), in a study aiming at comparing the efficacy of BDP vs. 5-ASA, employed enemas containing 3 mg in 100 ml.
- US 4,350,690 claims enema formulations containing between 0.1 to 2.0 mg in a volume from about 50 to 150 ml. Said formulations have been extemporary prepared and no stability data are reported.
- Kumana et al (Clin. Chem. 1981, 27, 1049; Lancet 1982, 1(8272), 579) employed enema formulations at low dose (1 mg) in order to avoid any systemic effects. According to the authors, said formulations are stable but it is not reported for how long.

US 5,378,470 claims pharmaceutical compositions for rectal use as dry material to be reconstituted immediately before use.

Bansky et al. (Dis Colon & Rectum 1987, 30(4), 288-292), for the same reason (i.e. to avoid systemic effects) utilise formulations containing 0.5 mg in 100 ml. Said formulations, comprising polysorbate 80, methyl *p*-hydroxy benzoate, sodium edetate and citric acid to pH 4.25, turned out to be stable for 12 months.

Vignotti et al. (Cur. Ther. Res. 52, 659 - 665, 1992) and D'Arienzo et al. (It. J. Gastroenterol & Hepatol 30, 254-257, 1998) reported on clinical efficacy of 60 ml enema containing 3 mg of BDP, but the characteristics of the formulation are not disclosed.

The enema formulation of the invention has optimized characteristics of viscosity in order to promote its retrograde progression and homogeneous distribution of the active ingredient on all the interested area; the effective therapeutical action is exerted locally, without inducing any significant side effects by keeping low the concentration of the active ingredient, in a small administration volume (3 mg in 60 ml).

It has been indeed found that xanthan gum is particularly effective in providing said optimized characteristics of viscosity, so that the active ingredient, upon rectal administration of the formulation, is able to reach, by retrograde progression, the sigmoid colon and the descending colon until to the splenic flexure and further up to the intestinal mucosa affected by the pathological process. In the known art, enema are generally not able to pass beyond the left side of the colon.

The pH and the salinity of the formulation of the invention have been also adjusted to physiological values, i.e. pH approximately 7 and osmolarity of about 280 mOsm/l, to make it well tolerated and to avoid further possible irritating effects against the inflamed mucosa.

Furthermore, the formulation of the invention turned out to be physically and chemically stable for at least three years, as it can be appreciated from the evaluation of the most significant chemical and technological parameters upon long-term storage conditions. This makes its use and the commercial distribution considerably easier.

The stability of the formulation of the invention at a pH of about 7 is an unexpected result. In the prior art (Foe et al. Biopharm. Drug Dispos. 19, 1-8, 1998), it is reported indeed that dissolved BDP in 0.002-0.004% phosphate buffer suspension at pH 7.4 undergoes a slow but considerable degradation due to hydrolysis. Said reaction could result, upon long-term periods as those envisaged for storing pharmaceutical formulations, in a decrease of the content which do not conform with the ICH (International Conference Harmonisation) requirements.

Finally, the formulation of the invention turned out to be stable without adding stabilizers such as anti-oxidants. It is well known indeed that some of the agents commonly utilised for this purpose may give rise to irritation or allergic reactions.

The invention is illustrated in greater detail by the following examples.

### Example 1. Preparation of 1000 bottles of BDP enemas

| Ingredients: | |
|---|---|
| Beclomethasone dipropionate | 30 g |
| Polysorbate 20 | 600 g |
| Sorbitan monolaurate | 120 g |
| Methyl parahydroxy benzoate | 1080 g |
| Propyl parahydroxy benzoate | 180 g |
| Benzyl alcohol | 3000 g |
| Xanthan gum | 3000 g |
| Cetostearyl alcohol | 780 g |
| Monobasic potassium phosphate | 2124 g |
| Dibasic sodium phosphate dehydrate | 4344 g |
| Sodium chloride | 2520 g |
| Purified water q.s. to | 600 l |

Methyl *p*-hydroxy benzoate and propyl *p*-hydroxy benzoate are added to water heated to 83÷88°C, and solubilised with the aid of a turbine. The solution is cooled down to 70÷75°C, cetostearyl alcohol, polysorbate 20 and sorbitan monolaurate, benzyl alcohol, potassium phosphate salts, sodium phosphate and sodium chloride are added, and the resulting suspension is stirred. The preparation is cooled down to 42÷45°C, than the active ingredient is added under stirring.. The xanthan gum is dispersed, under stirring. The final volume is controlled, adding water if necessary. The suspension is stirred under vacuum and cooled down to room temperature. The pH is approximately 7.0.

### Example 2. Evaluation of the retrograde progression of the enemas.

In order to assess the capability of the medicament to reach the inflammation site, a test was performed using a scintigraphic technique well known in literature, which is suitable to detect the extension and limits of the retrograde progression of BDP enemas.

BDP rectal enema was radiolabeled with technetium 99 m (⁹⁹Tc^{m}) colloidal sulfide and administered in single 3 mg dose to 8 adult patients with distal ulcerative colitis moderately active or in remission. Scintigraphic recordings were then carried out at 5, 30, 60, 120, 180, 210 and 240 minutes after dosing, with patients lying face upwards. Total radioactivity (COU), pixel number (CELL) and mean radioactivity per pixel (AVG) in the different intestinal portions were evaluated.

The resulting data showed a significant difference of the "retrograde spread" of BDP enemas in patients with active distal ulcerative colitis and in remission patients. Whereas in the latter the upward progression of the radiolabelled solution stopped at the first tracts of the intestine, in the patients with active ulcerative colitis the retroprogression of BDP enemas was much more extended, reaching, in some cases, the hepatic flexure, thus meeting the expected requirements.

### Example 3. Evaluation of the absorption

In order to demonstrate that no systemic absorption occurred, BDP and its major active metabolites, Beclomethasone-17-monopropionate (B17MP) and Beclomethasone (BOH), were determined in plasma and urine samples in subjects undergoing treatment with the formulations of the invention.

A first study was performed in 9 healthy male volunteers, after a single administration of enemas by rectal route. No detectable amounts of the drug and its metabolites were found on plasma and urine samples, indicating that, upon rectal administration, the BDP activity is exerted only topically.

A subsequent study carried out in 10 ileostomized patients, 5 of whom treated with 5 mg modified-release BDP coated tablets and the other 5 with 5 mg BDP uncoated tablets, confirmed the absence of the active ingredient and of its metabolites both in plasma and urine samples collected during the study. The analysis of intestinal effluates showed the presence of significant amounts of BDP and B17MP, indicating that a significant release of active ingredient occurred at the action site, i.e. the distal part of the ileum.

The absence of systemic absorption of unmetabolised BDP and/or of its metabolites was also assessed in a pharmacokinetic-scintigraphic study performed in healthy volunteers (example 4, § "Disintegration"), to further confirm the topical action of the compound.

### Example 4. Stability of enemas BDP formulation

The long-term stability (21-24°C, 52-60% R.H.) was evaluated after storing the formulation in polyethylene flasks. The results are reported in Table 1. The BDP content was determined by HPLC. The other following parameters were monitored: appearance, pH, viscosity and the microbial count according to Ph. Eur. III Ed.

In said conditions, the formulation turned out to be stable for at least three years.

**Table 1**

| Parameter | Appearance | pH | Viscosity (cp) | BDP Content (%) | Microbial count |
|---|---|---|---|---|---|
| Time : 0 | Homogeneous | 6.97 | 800 | 100 | complies |
| : 6 months | unchanged | 6.96 | 810 | 104.0 | n.d. |
| : 12 months | unchanged | 6.95 | 860 | 103.3 | n.d. |
| : 18 months | unchanged | 6.95 | 815 | 98.5 | n.d. |
| : 24 months | unchanged | 6.56 | 800 | 98.5 | n.d. |
| : 36 months | unchanged | 6.48 | 795 | 100.4 | complies |
| **n.d. not determined** | | | | | |

## Claims

1. A pharmaceutical formulation for the treatment of inflammatory bowel disease in the form of ready-to-use enema, comprising, suspendenl in 60 ml of an aqueous solution, 3 mg beclomethasone dipropionate as active ingredient and 300 mg xanthan gum as suspending and thickening agent, and having an osmolarity of about 280 mOsml/l.

2. A pharmaceutical formulation according to claim 1, further comprising sodium chloride as an agent for adjusting salinity.

3. A pharmaceutical formulation according to claim 1, further comprising antimicrobials, buffering salts and surfactants and/or wetting agents.

4. A pharmaceutical formulation according to claim 2, wherein the antimicrobials are selected from benzyl alcohol, methyl and propyl *para*-hydroxybenzoates.

5. A pharmaceutical formulation according to claim 2 wherein the surfactants and/or wetting agents are selected from cetostearyl alcohol, polysorbate 20 and sorbitan monolaurate.

## Patentansprüche

1. Pharmazeutische Formulierung zur Behandlung von entzündlicher Darmerkrankung in der Form eines gebrauchsfertigen Einlaufs, umfassend, suspendiert in 60 ml einer wässrigen Lösung, 3 mg Beclomethason-Dipropionat als aktives Ingredienz und 300 mg Xanthangummi als Suspendier- und Verdickungsmittel, der eine Osmolarität von etwa 280 mOsml/l hat.

2. Pharmazeutische Formulierung nach Anspruch 1, die außerdem Natriumchlorid als ein Mittel zur Einstellung der Salinität umfasst.

3. Pharmazeutische Formulierung nach Anspruch 1, die außerdem antimikrobielle Mittel, puffernde Salze und oberflächenaktive Mittel und/oder Netzmittel umfasst.

4. Pharmazeutische Formulierung nach Anspruch 2, wobei die antimikrobiellen Mittel ausgewählt sind aus Benzylalkohol, Methyl- und Propyl-*para*-hydroxybenzoaten.

5. Pharmazeutische Formulierung nach Anspruch 2, wobei die oberflächenaktiven Mittel und/oder Netzmittel ausgewählt sind aus Cetostearylalkohol, Polysorbat 20 und Sorbitanmonolaurat.

## Revendications

1. Formulation pharmaceutique pour le traitement d'une maladie intestinale inflammatoire, sous la forme d'un lavement prêt à l'emploi, comprenant, en suspension dans 60 ml d'une solution aqueuse, 3 mg de dipropionate de béclométhasone en tant qu'ingrédient actif et 300 mg de gomme xanthane en tant qu'agent de suspension et épaississant, et ayant une osmolarité d'environ 280 mOsml/l.

2. Formulation pharmaceutique selon la revendication 1, comprenant en outre du chlorure de sodium en tant qu'agent pour ajuster la salinité.

3. Formulation pharmaceutique selon la revendication 1, comprenant en outre des antimicrobiens, des sels tamponnants et des agents tensioactifs et/ou des agents mouillants.

4. Formulation pharmaceutique selon la revendication 2, dans laquelle les antimicrobiens sont choisis parmi l'alcool benzylique, les *para*-hydroxybenzoates de méthyle et de propyle.

5. Formulation pharmaceutique selon la revendication 2, dans laquelle les agents tensioactifs et/ou les agents mouillants sont choisis parmi l'alcool cétostéarylique, un polysorbate 20 et le monolaurate de sorbitane.
